# EUROPÄISCHE PATENTSCHRIFT

(11) **EP 0 415 442 B1**
(45) Veröffentlichungstag und Bekanntmachung des Hinweises auf die Patenterteilung: **07.09.1994**
(21) Anmeldenummer: 90116760.1
(22) Anmeldetag: 31.08.1990
(51) Int. Cl.: G01N 33/53, G01N 33/543, G01N 33/74

(54) **Verfahren zum Nachweis von Analyten**
Method for detecting an analyte
Méthode pour la détection d'un analyte

(30) Priorität: 01.09.1989 DE 3929119
(43) Veröffentlichungstag der Anmeldung: 06.03.1991
(73) Patentinhaber: BOEHRINGER MANNHEIM GMBH, 68298 Mannheim (DE)
(72) Erfinder: Baumgarten, Horst, Dr., D-8122 Penzberg (DE); Grol, Michael, Dr., D-8133 Feldafing (DE); Stahl, Peter, Dr., D-8131 Bernried (DE)
(74) Vertreter: Huber, Bernhard, Dipl.-Chem.

(56) Entgegenhaltungen:
- EP-A- 0 045 103
- EP-A- 0 322 813
- WO-A-83/04312
- WO-A-87/06004
- US-A- 4 471 058
- SCIENCE, vol. 221, 15 July 1983; P.H. EHRLICH et al., pp. 279-281#

## Beschreibung

Die Erfindung betrifft ein Verfahren zum Nachweis von Analyten mit immunologischen Verfahren.

In der klinischen Diagnostik haben Nachweismethoden nach dem Prinzip des Immunoassays eine große Bedeutung erlangt. Mit immunologischen Verfahren lassen sich sehr viele Substanzen genau und spezifisch nachweisen. Hierzu sind Antikörper erforderlich, die sehr spezifisch mit der nachzuweisenden Substanz, nicht aber mit ähnlich strukturierten Verbindungen Komplexe bilden. Das Auffinden spezifischer Antikörper ist daher ein sehr wesentlicher Teil bei der Erarbeitung neuer Nachweisverfahren bzw. der Verbesserung bekannter Verfahren. Eine erhebliche Verbesserung brachte das Verfahren von Koehler und Milstein, mit dem monoklonale Antikörper produziert werden können. Während die Produktion von Hybridoma-Zellinien, die monoklonale Antikörper produzieren, an sich keine Schwierigkeiten mehr bietet, ist das Screeningverfahren, mit dem die jeweils geeigneten Zellinien, die spezifische und hochaffine Antikörper produzieren, ausgewählt werden, oft aufwendig. Je höher die Anforderungen an die Eigenschaften des monoklonalen Antikörpers sind, desto höher wird auch der Screeningaufwand und unter Umständen ist es gar nicht möglich, einen passenden einzelnen monoklonalen Antikörper zu finden. Die Hauptanforderungen, die an einen Antikörper gestellt werden, sind einerseits eine hohe Affinität, mit der der Antikörper an "sein" Antigen oder Hapten bindet, und andererseits eine große Spezifität, mit der der Antikörper "sein" Antigen oder Hapten erkennt, und damit niedrige Kreuzreaktivität. Die Affinität des Antikörpers richtet sich nach den jeweiligen Gegebenheiten, z.B. Analytkonzentration. Die Kreuzreaktivität des gesuchten Antikörpers zu "anderen" Antigenen soll unterhalb von bestimmten Werten liegen. Diese Werte werden so festgelegt, daß diese Antigene keine über die Fehlergrenze hinausgehende Änderung des Meßsignals in dem jeweiligen Nachweisverfahren bewirken.

Oft ergibt die Suche nach einem bezüglich Affinität und Spezifität idealen monoklonalen Antikörper keinen einzigen testtauglichen Antikörper, jedoch einige, die die Anforderungen in den meisten, aber nicht allen Punkten erfüllen.

Das Verfahren nach dem Stand der Technik besteht darin, die Testbedingungen (pH, Salzkonzentration, Temperatur, etc.) so zu verändern, bis mit dem verwendeten monoklonalen Antikörper Kreuzreaktivitäten erhalten werden, die für einen klinischen Test tolerabel sind. Dieses Verfahren ist jedoch zeitaufwendig und führt oft nicht zu dem gewünschten Ergebnis.

Es war nun Aufgabe der Erfindung, kompetitive immunologische Nachweisverfahren zu verbessern, durch Bereitstellung von Rezeptoren mit höherer Spezifität.

Diese Aufgabe wird gelöst durch ein Verfahren zum immunologischen Nachweis von monovalenten Analyten durch Konkurrenz des zu bestimmenden Analyten mit einer bekannten Menge an markiertem oder immobilisiertem Analyten um die Bindungsstelle eines Rezeptors für den Analyten, das dadurch gekennzeichnet ist, daß man als Rezeptor eine Mischung von mindestens zwei mit dem Analyten spezifisch bindefähigen monoklonalen Antikörpern, deren Kreuzreaktivität verschieden ist, verwendet.

Überraschenderweise gelingt es durch Verwendung einer Mischung monoklonaler Antikörper, die jeder für sich die Anforderungen an Spezifität und Affinität nicht erfüllen, ein Testsystem zur Verfügung zu stellen, dessen Spezifität gegenüber den Einzelsubstanzen verbessert ist.

Für kompetitive immunologische Nachweisverfahren gibt es viele Varianten, die allgemein bekannt sind. In der Regel werden für monovalente Analyten, insbesondere Haptene, zwei Testprinzipien bevorzugt: Entweder reagiert der Rezeptor, der an eine Festphase gebunden ist, mit der Probe und dem markierten Analyten, oder ein markierter Rezeptor reagiert mit der Probe und einem an die Festphase gebundenen Analyten. Bei dem die Erfindung betreffenden Verfahren wird der Antikörper in der Regel in äquimolarer Menge oder im Unterschuß eingesetzt.

Die gebildeten Komplexe können abgetrennt werden und in bekannter Weise die Markierung ausgewertet werden. Für alle Verfahrensvarianten bringt das erfindungsgemäße Verfahren eine Verbesserung.

Abhängig von dem jeweiligen Verfahren kann die erfindungsgemäße Antikörpermischung als markierter Rezeptor, festphasengebundener Rezeptor, agglutinierender Rezeptor u.s.w. eingesetzt werden. Für die erfindungsgemäß verwendete Antikörpermischung werden monoklonale Antikörper ausgewählt, deren Affinität im optimalen Bereich liegt und die wenige Kreuzreaktionen aufweisen, wobei die Kreuzreaktivität über dem Sollwert liegt. Die Kreuzreaktivitäten der für die Mischung verwendeten Antikörper sind verschieden, d.h. die Antikörper reagieren mit verschiedenen der nachzuweisenden Substanz ähnlichen Verbindungen. Anders ausgedrückt geht der eine Antikörper Kreuzreaktionen mit Substanzen ein, mit denen der andere Antikörper nicht oder praktisch nicht nachweisbar reagiert (und umgekehrt). Die Kreuzreaktivität eines Antikörpers zu einer bestimmten Substanz ist dabei keine absolute, sondern eine relative Meßgröße, die vom jeweiligen Testsystem abhängt. Bezugspunkt ist der zu bestimmende Analyt, an den der Antikörper spezifisch bindet. Das Meßsignal, das bei der Reaktion mit dem Analyten erreicht wird, wird zu 100 % gesetzt. In Abwesenheit oder Gegenwart von Analyt werden nun verschiedene Konzentrationen der kreuzreagierenden Substanz zur Testlösung gegeben. Mit der kreuzreagierenden Substanz wird dann ein Meßsignal erhalten, das einer bestimmten Menge des eigentlichen Analyten entspricht. Abgelesen wird im Bereich der höchsten Präzision der Eichkurve. Dieses Signal wird ebenfalls in Prozent ausgedrückt. Eine Kreuzreaktion von z.B. 10 % bedeutet also, daß man die 10fache Menge einer kreuzreagierenden Substanz einsetzen muß, um dasselbe Meßsignal wie mit der einfachen Menge des Analyten zu erreichen. In einem anderen Testsystem kann für die kreuzreagierende Substanz ein anderer Wert ermittelt werden. Die in einem Test geforderten Kreuzreaktionen (Sollwerte) sind abhängig von dem Konzentrationsverhältnis der jeweiligen kreuzreagierenden Substanz zum Analyten und können deshalb sehr unterschiedlich sein.

Für das erfindungsgemäße Verfahren können monoklonale Antikörper verwendet werden, deren Kreuzreaktivität bezüglich einer Substanz das bis zu Dreifache, bevorzugt bis zu Zweifache des oberen Sollwertes beträgt. Der Soll-Bereich liegt zwischen der Nachweisgrenze und der für den jeweiligen Test akzeptierbaren Fehlergrenze. Erfindungsgemäß wird eine Mischung von mindestens zwei monoklonalen Antikörpern verwendet. Es können auch mehr als 2 monoklonale Antikörperarten gemischt werden. Das Verhältnis, in dem die einzelnen monoklonalen Antikörper in der Mischung vorliegen, richtet sich nach ihrer Affinität und Spezifität. In der Regel wird der Antikörper mit der geringsten Kreuzreaktivität den größten Anteil liefern. Das Verhältnis der einzelnen Antikörper wird nach den Gegebenheiten ausgewählt. Bevorzugt wird eine Mischung von zwei Antikörpern eingesetzt, deren Verhältnis im Bereich von 0,1 bis 10:1 liegt.

Durch die Mischung von mindestens zwei Antikörpern, die mit verschiedenen Verbindungen störende Nebenreaktionen eingehen, wird die Kreuzreaktivität insgesamt gesenkt. Diese Absenkung geht dabei in der Regel über einen Verdünnungseffekt hinaus, so daß offensichtlich eine synergistische Wirkungssteigerung vorliegt.

Das erfindungsgemäße Verfahren ermöglicht es, monoklonale Antikörper einzusetzen, die hinsichtlich der Spezifität nicht ideal sind. Das Screening auf solche, nur partiell testtauglichen Antikörper ist weniger risikoreich und daher billiger und schneller. Durch die Verwendung einer Mischung von zwei oder mehreren monoklonalen Antikörpern, die jeder für sich alleine nicht testtauglich sind, können ausreichend spezifische Testsysteme zur Verfügung gestellt werden, da durch die Mischung von mehreren Antikörpern mit verschiedener Kreuzreaktivität insgesamt ein System zur Verfügung gestellt werden kann, das den Anforderungen genügt.

Die Erfindung wird durch die folgenden Figuren und Beispiele erläutert.
- Fig. 1: zeigt ein Diagramm, in das für verschiedene Mischungen der beiden Antikörper 19.6.9 und 1.26.18 erhaltene Kreuzreaktivitäten (normiert) eingetragen sind;
- Fig. 2: zeigt ein Diagramm, in das für verschiedene Mischungen der beiden Antikörper 19.6.9 und 9B4.C2 erhaltene Kreuzreaktivitäten (normiert) eingetragen sind;
- Fig. 3: zeigt ein Diagramm, in das für verschiedene Mischungen der beiden Antikörper 8.15.1 und 700-03 erhaltene Kreuzreaktivitäten (normiert) eingetragen sind.
- Fig. 4: zeigt ein Diagramm, in das für verschiedene Mischungen der beiden Antikörper 2.341.115 und 2.93.37 erhaltene Kreuzreaktivitäten (normiert) eingetragen sind.

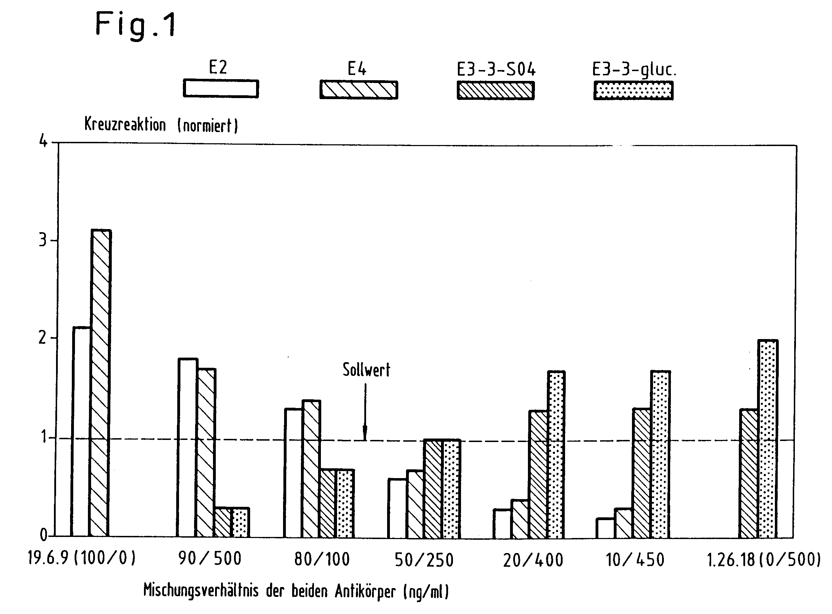

Da klinisch relevante Kreuzreaktionen sehr unterschiedlich sein können, z.B. < 0,1% oder < 20%, wird eine graphische Darstellung bei Angabe in Prozentwerten erschwert. Um dies zu umgehen, wird in den Figuren 1 bis 3 die jeweilig maximale XR (Kreuzreaktivität) eines Antikörpers zu 1 gesetzt (= normierte XR). Die gestrichelte Linie stellt die errechnete (erwartete) XR beim Volumenmischungsverhältnis 1 : 1 dar.

### Beispiel 1

Bestimmung von Steroidkonzentrationen in Lösungen (Testprinzip)

Zum Nachweis von Steroiden in Puffer oder Körperflüssigkeiten (Serum, Plasma, Urin, etc.) werden Enzymtests nach dem kompetitiven Prinzip verwendet:

**Materalien:**
- Unbeschichtete Luran Tubes
- Streptavidin
- Rinderserumalbumin (RSA)
- Substratlösung:
   (100 mmol/l Phosphat-Citrat-Puffer
   pH 4,4
   3,2 mmol/l Natriumperborat
   1,9 mmol/l ABTS (2,2′-Azino-di-[3-ethyl-benzthiazolinsulfonsäure(6)]-diammoniumsalz)
- Beschichtungspuffer:
   200 mmol/l Natriumcarbonatpuffer
   pH 7,2
- Verdünnungspuffer :
   50 mmol/l Natriumphosphatpuffer
   pH 6,8, 0,3 % RSA
- Waschlösung :
   0,9% NaCl

Die Biotinylierung der MAKs erfolgt gemäß JACS 100 (1978), 3585-3590 mit Biotin durch Umsetzung mit N-Hydroxysuccinimid-biotin im Verhältnis 10:1.
a) Bestimmung mit direkt beschichteten Tubes
   Die Tubes werden mit einer Präparation einer gereinigten anti-Antikörperlösung (10 µg/ml) in einem Beschichtungspuffer (0,2 mol/l Natriumcarbonat/-Bicarbonat, pH 7,2) bei Raumtemperatur für 1 Stunde inkubiert. Es erfolgt eine spontane Bindung dieses Antikörpers an die Tubewand. Die Tubes werden dann mit 50 mmol/l Phosphatpuffer/0,3 % Albumin für 15 Minuten nachbehandelt und gewaschen.
   Die spezifischen Antikörper werden in Verdünnungspuffer zugegeben und für 60 Minuten inkubiert. Danach werden 600 µl Probe (Analyt, kreuzreagierende Substanz etc.) und 500 µl Enzymkonjugat (Östriol-POD, POD-Aktivität: 200 mU/ml) zugegeben und für 60 Minuten inkubiert. Um die Kreuzreaktion zu anderen Steroiden zu überprüfen, werden der Probelösung unterschiedliche Mengen des zu prüfenden Steroids zugesetzt. Eine Erniedrigung des Meßsignals durch andere Steroide bedeutet eine Kreuzreaktion, deren Höhe an der Standardkurve mit dem eigentlichen Analyten abgelesen werden kann. Nach einem weiteren Waschschritt wird die Peroxidaseaktivität durch Zusatz von Substratlösung bestimmt, zur Entwicklung des Farbstoffs (1000 µl) erfolgt vorher eine weitere Inkubation (30 Min.). Die Messung des Farbumsatzes erfolgt bei 422 nm im Photometer.
b) Bestimmung mit Streptavidin-beschichteten Tubes
   Die Tubes werden mit einer Streptavidinlösung 1 Stunde bei Raumtemperatur in Beschichtungspuffer inkubiert und mit 50 mmol/l Phosphatpuffer/0,3% Albumin für 15 Minuten nachbehandelt und gewaschen.
   Die spezifischen Antikörper werden in Verdünnungspuffer zugegeben und für 60 Minuten inkubiert. Danach werden 600 µl Probe (Analyt, kreuzreagierende Substanz etc.) und 500 µl Enzymkonjugat (Östriol-POD, POD-Aktivität: 200 mU/ml) zugegeben und für 60 Minuten inkubiert. Um die Kreuzreaktion zu anderen Steroiden zu überprüfen, werden der Probelösung unterschiedliche Mengen des zu prüfenden Steroids zugesetzt. Eine Erniedrigung des Meßsignals durch andere Steroide bedeutet eine Kreuzreaktion, deren Höhe an der Standardkurve mit dem eigentlichen Analyten abgelesen werden kann. Nach einem weiteren Waschschritt wird die Peroxidaseaktivität durch Zusatz von Substratlösung bestimmt, zur Entwicklung des Farbstoffs (1000 µl) erfolgt vorher eine weitere Inkubation (30 Min.). Die Messung des Farbumsatzes erfolgt bei 422 nm im Photometer.

### Beispiel 2

Bestimmung von Kreuzreaktionen für Mischungen von MAK<Östriol>19.6.9 und MAK<Östriol>1.26.18

**Materalien:**
- E3 POD-Konjugat (Das E3-POD-Konjugat wird hergestellt, indem Östriol über die 6-Position mit Hemisuccinat aktiviert und anschließend mit Succinimid verestert wird. Der entstandene Ester wird direkt an Peroxidase gekoppelt) (500 und 100 mU POD-Aktivität/ml)
- MAK<E3>19.6.9 (ECACC 89082503) biotinyliert (100 µg/ml)
- MAK<E3>1.26.18 (ECACC 89082502) biotinyliert (1000 µg/ml)

Konzentrationen der eingesetzten kreuzreagierenden Substanzen
- Östriol (E3): : 0, 5, 10, 20, 40, ng/ml (im Test 1:5 verd.)
- Östradiol (E2): : 10, 100 ng/ml
- Östetrol (E4): : 10, 100 ng/ml
- E3-3-sulfat: : 50, 100 ng/ml
- E3-3-glucuronid: : 50, 100 ng/ml

Die Durchführung der Bestimmung erfolgt nach Beispiel 1b.

Die Ergebnisse (Fig. 1) zeigen, daß MAK 19.6.9 stark mit Östradiol und Östetrol kreuzreagiert, während MAK 1.26.18 stark mit Östriol-3-sulfat und Östriol-3-glucuronid kreuzreagiert. Die Antikörper wurden in verschiedenen Mischungsverhältnissen eingesetzt. Bei verschiedenen Mischungsverhältnissen ändern sich die jeweiligen Kreuzreaktionen. Es ergibt sich ein optimaler und ein bevorzugter Bereich.

### Beispiel 3

Bestimmung von Kreuzreaktionen für Mischungen von MAK<Östriol>19.6.9 und MAK<Östriol>9B4.C2

Die Durchführung der Bestimmung erfolgt analog Beispiel 1a. MAK 9B4.C2 (Hersteller IPL, Interpharm Laboratories Ltd., Ness Ziona 7611O, Israel) reagiert (analog dem 1.26.18) stark kreuz mit E3-3-sulfat und E3-3-glucuronid.

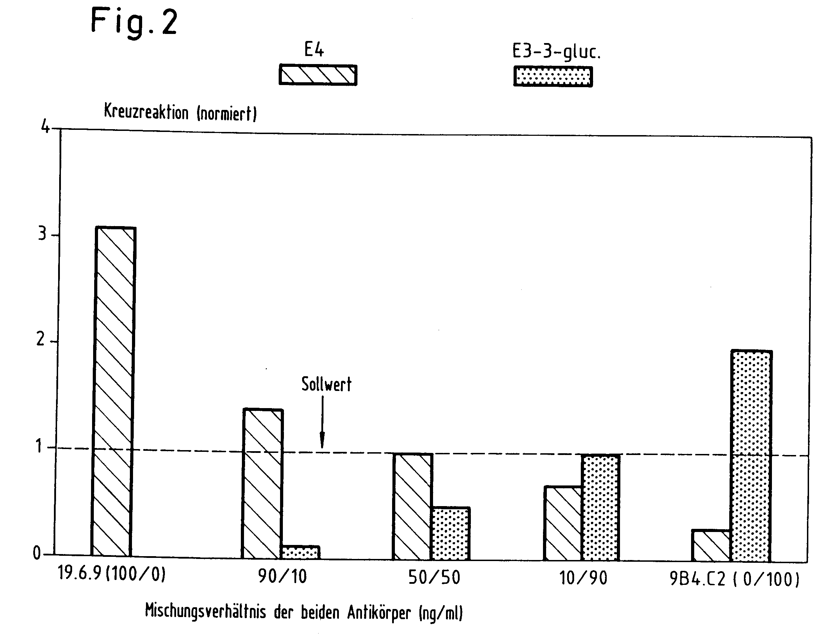

Auch hier gibt es eine MAK-Konzentration, bei der die XR deutlich reduziert ist (s. Fig. 2).

### Beispiel 4

Bestimmung der Kreuzreaktionen für Mischungen von MAK<Testosteron>8.15.1 und MAK<Testosteron>700-O3
Materalien:
- Unbeschichtete Luran Tubes
- polyklonaler Schafantikörper (IgG) gegen Maus-Fcγ (10 µg/ml; zur Beschichtung der Tubes)
- Crotein C
- Testosteron POD-Konjugat (Das Testosteron-POD-Konjugat wird hergestellt, indem Testosteron über die 3-Position mit Carboxymethyloxin aktiviert und mit Succinimid verestert wird. Der entstandene Ester wird direkt an die POD gekoppelt (POD-Aktivität 100 mU/ml)
- Testosteron
- 4-Androsten-3,17-dion = Androstendion
- 11-keto-Testosteron
- MAK<Testosteron>8.15.1 (ECACC 89082501)
- MAK<Testosteron>700-03 (Hersteller: Medix Biotech Inc., Forster City, CA 944O4, USA; Best.-Nr. T-700-03) Konzentrationen der eingesetzten kreuzreagierenden Substanzen:

- Testosteron: : 0, 1.25, 2.5, 5, 10, 20, 40, ng/ml
- Androstendion: : 2.5, 5, 10, 20, 40, 80, 160, 320, 640 ng/ml
- 11-keto-Testosteron: : 2.5, 5, 10, 20, 40, 80, 160, 320, 640 ng/ml

Die Durchführung der Bestimmung erfolgt analog Beispiel 1a

### Ergebnis:

MAK 8.15.1 und MAK 700-03 sind spezifische Antikörper gegen Testosteron, weisen aber relativ hohe XR zu Androstendion resp. 11-keto-Testosteron auf.

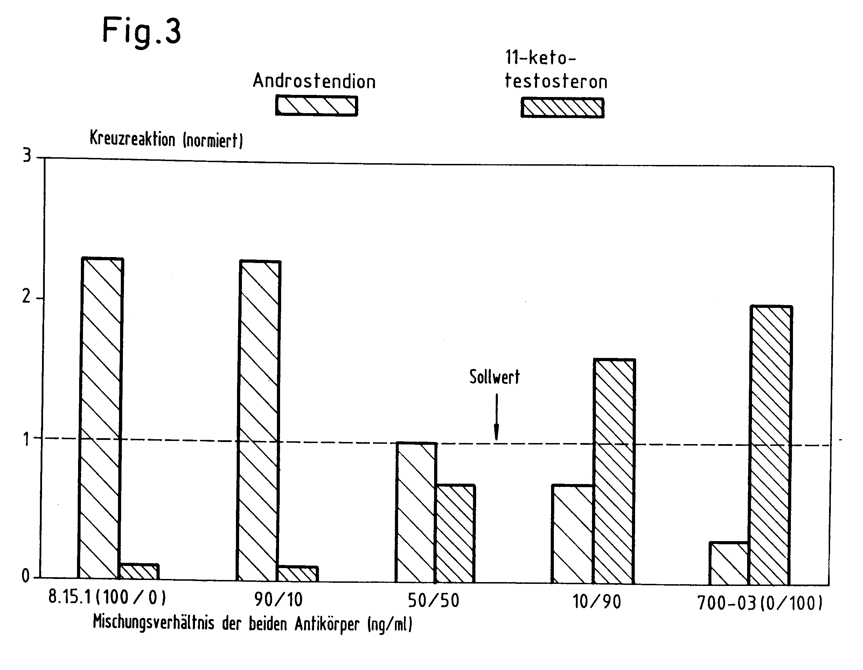

Die nicht gekoppelten Antikörper wurden in verschiedenen Mischungsverhältnissen eingesetzt (vgl. Fig. 3).

Die XR der MAK-Mischung gegen Testosteron läßt sich durch Mischen beeinflussen, auch hier läßt sich ein Mischungsverhältnis finden, bei dem alle Kreuzreaktionen unter den erwarteten Werten liegen (Fig. 3)

### Beispiel 5

Bestimmung der Kreuzreaktion für Mischungen von
MAK<Phenobarbital>2.341.115 und
MAK<Phenobarbital>2.93.37

**Materialien:**
- Unbeschichtete Luran Tubes
- polyklonaler Schafantikörper (IgG) gegen Maus-Fcγ (10 ug/ml; zur Beschichtung der Tubes)
- Crotein C
- Phenobarbital-POD-Konjugat (Das Phenobarbital-POD-Konjugat wird hergestellt, indem aus Phenobarbital über N in 1-Stellung mit Butyrat aktiviert und mit Succinimid verestert wird. Der entstandene Ester wird direkt an die POD gekoppelt(POD-Aktivität 100 mU/ml).
- Phenobarbital
- Secobarbital
- Amobarbital
- Pentobarbital
- Primidon
- MAK<Phenobarbital>2.341.115 (ECACC 90071903)
- MAK<Phenobarbital>2.93.37 (ECACC 90071904)

Konzentration der eingesetzten kreuzreagierenden Substanzen:
- Phenobarbital: : 0, 7.8, 15.6, 31.3, 62.5, 125 ng/ml
- Secobarbital: : 0.2, 1 µg/ml
- Amobarbital: : 0.2, 1 µg/ml
- Pentobarbital: : 0.2, 1 µg/ml
- Primidon: : 1, 5 µg/ml

Die Durchführung der Bestimmung erfolgt analog Beispiel 1a.

### Ergebnis:

MAK 2.341,115 und MAK 2.93.37 sind spezifische Antikörper gegen Phenobarbital, weisen aber relativ hohe Kreuzreaktionen einerseits zu Primidon (MAK 2.341.115) andererseits zu Secobarbital, Amobarbital und Pentobarbital (MAK 2.93.37) auf.

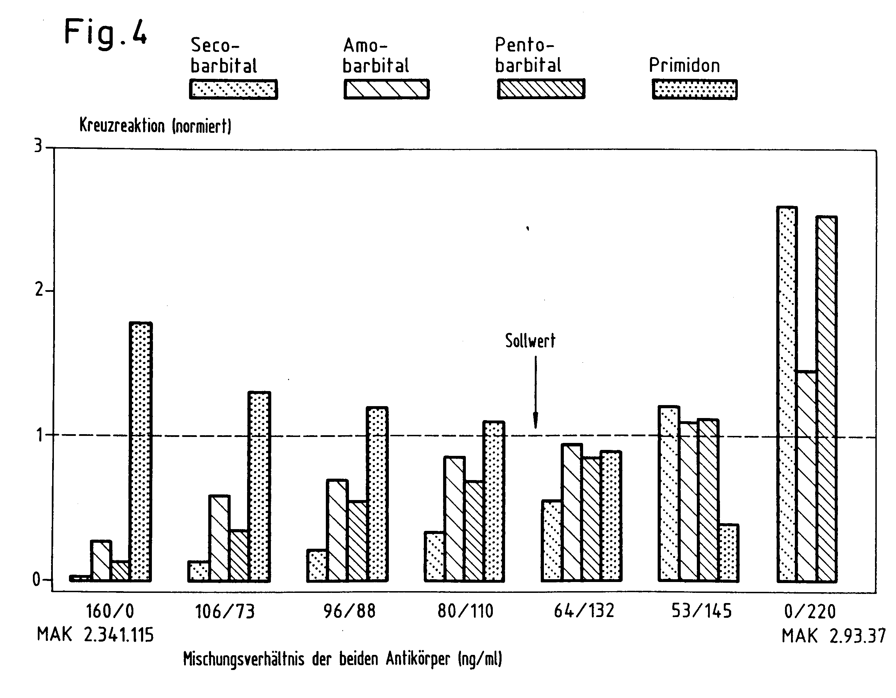

Die Antikörper werden in verschiedenen Mischungsverhältnissen eingesetzt (vgl. Fig. 4).

Die Kreuzreaktion der MAK-Mischung gegen Phenobarbital läßt sich durch Mischen beeinflussen. Auch hier läßt sich ein Mischungsverhältnis finden, bei dem alle Kreuzreaktionen unter den erwarteten Werten liegen (Fig. 4)

## Patentansprüche

1. Verfahren zum immunologischen Nachweis von monovalenten Analyten durch Konkurrenz des zu bestimmenden Analyten mit einer bekannten Menge an markiertem oder immobilisiertem Analyten um die Bindestelle eines Rezeptors für den Analyten,
**dadurch gekennzeichnet,**
daß man als Rezeptor eine Mischung von mindestens zwei mit dem Analyten spezifisch bindefähigen monoklonalen Antikörpern, deren Kreuzreaktivität verschieden ist, verwendet.

2. Verfahren nach Anspruch 1,
**dadurch gekennzeichnet,**
daß eine Mischung von zwei monoklonalen Antikörpern in einem Verhältnis von 0,1 bis 10:1 verwendet wird.

3. Verfahren nach Anspruch 1 oder 2,
**dadurch gekennzeichnet,**
daß monoklonale Antikörper verwendet werden, die Kreuzreaktivität mit ein oder zwei Substanzen aufweisen, die in einem Bereich zwischen der Nachweisgrenze und dem Dreifachen der für den jeweiligen Test annehmbaren Grenze liegt.

## Claims

1. Method for the immunological detection of monovalent analytes by competition of the analyte to be determined with a known amount of labelled or immobilized analyte for the binding site of a receptor for the analyte,
**wherein**
a mixture of at least two monoclonal antibodies which are capable of specific binding to the analyte and which have different cross-reactivities are used as the receptor.

2. Method as claimed in claim 1,
**wherein**
a mixture of two monoclonal antibodies in a ratio of 0.1 to 10:1 is used.

3. Method as claimed in claims 1 or 2,
**wherein**
monoclonal antibodies are used which have a cross-reactivity with one or two substances which is in a range between the detection limit and three times the acceptable limit for the respective test.

## Revendications

1. Procédé de détection immunologique d'analytes monovalents par compétition de l'analyte à déterminer avec une quantité connue d'analyte marqué ou immobilisé pour le site de liaison d'un récepteur pour l'analyte, caractérisé en ce que l'on utilise comme récepteur un mélange d'au moins deux anticorps monoclonaux capables de se lier de manière spécifique à l'analyte, dont la réactivité croisée est différente.

2. Procédé selon la revendication 1, caractérisé en ce que l'on utilise un mélange de deux anticorps monoclonaux dans un rapport de 0,1 à 10:1.

3. Procédé selon la revendication 1 ou 2, caractérisé en ce que l'on utilise des anticorps monoclonaux qui présentent une réactivité croisée avec une ou deux substances qui est située dans un domaine entre la limite de détection et le triple de la limite acceptable pour le test correspondant.
